# EUROPEAN PATENT APPLICATION

(11) **EP 4 663 203 A1**
(43) Date of publication of application: **17.12.2025**
(21) Application number: 23920391.2
(22) Date of filing: 08.02.2023
(51) Int. Cl.: A61K 45/06, A61K 31/045, A61K 31/351, A61P 9/04

(54) **COMBINATION OF SGLT2 INHIBITOR AND MENTHOL AND USE THEREOF IN TREATMENT OF HEART DISEASE**

(71) Applicant: East China University of Science and Technology, Shanghai 200237 (CN)
(72) Inventor: LI, Jian, Shanghai 200237 (CN); XU, Yixiang, Shanghai 200237 (CN); HUANG, Yunyuan, Shanghai 200237 (CN); ZHANG, Chao, Shanghai 200237 (CN); YAO, Yue, Shanghai 200237 (CN)
(74) Representative: dompatent
(86) International application number: PCT/CN2023/075028
(87) International publication number: WO 2024/164183

(57) **Abstract**

Disclosed in the present invention are a pharmaceutical composition of an SGLT2 inhibitor and menthol and a use thereof in the preparation of drugs for preventing, ameliorating, and/or treating heart diseases. The pharmaceutical composition involved in the present invention has a myocardial cell protection effect, synergistically reduces intracellular calcium ion concentration, synergistically promotes fatty acid metabolism, has advantages compared with a single medication, and can be used for preparing medicines for treating, ameliorating, and/or preventing heart failure, coronary heart disease, myocardial infarction, hypertension heart disease, etc.

## Description

### Technical Field

The present invention relates to the field of pharmacotherapy, and in particular to a pharmaceutical composition of an SGLT2 inhibitor and menthol, in particular a combination of empagliflozin and L-menthol, for use in preventing and/or treating heart diseases.

### Background Art

Heart disease is a relatively common circulatory system disease. It is divided into many types according to different pathological mechanisms, mainly including heart failure, coronary heart disease, myocardial infarction, myocarditis, myocardial disease, hypertensive heart disease, arrhythmia, dilated cardiomyopathy, etc. Among them, heart failure is the terminal stage of many heart diseases. Heart failure, abbreviated as HF, is a group of clinical diseases characterized by insufficient tissue perfusion caused by impaired cardiac systolic and/or diastolic function, and reduced ejection and filling capacity. Its common clinical symptoms are dyspnea, general fatigue and weakness, accompanied by signs such as systemic circulation/pulmonary circulation congestion and peripheral edema. As a late clinical symptom of various cardiovascular diseases, the morbidity, hospitalization rate and mortality rate caused by heart failure are extremely high, and it has become the main cause of hospitalization for people over 65 years old. According to statistics, the number of heart failure patients worldwide has reached 23 million, among which the morbidity of heart failure in developed countries is about 1-2%. The situation in our country is not optimistic either. According to the 2015 Heart Failure Epidemiological Survey, the weighted heart failure prevalence among residents aged ≥ 35 years in my country is 1.3%, which means that approximately 13.7 million people suffer from heart failure, and the trend continues to rise.

Heart failure is a complex disease process caused by multiple pathogenic factors. However, neuro-humoral stimulation plays the most important role in the occurrence and development of heart failure. Among them, long-term overactivation of the renin-angiotensin-aldosterone system (RAAS) and the sympathetic nervous system (SNS) can lead to excessive vasoconstriction, increase peripheral resistance, increase cardiac afterload, and induce dysfunction, leading to pathological myocardial hypertrophy, fibrosis, adverse ventricular remodeling, and ultimately heart failure. In addition, with the occurrence and development of heart failure, the synthesis and release of factors such as natriuretic peptides, arginine vasopressin, TNF-α, and interleukins increase, which can also cause myocardial cell apoptosis and ventricular remodeling by affecting blood volume and inflammatory response, aggravating heart failure; impaired myocardial energy metabolism results in insufficient cardiac energy production to maintain normal work demands, resulting in dysfunction of the heart's pumping function and ion transport function, further aggravating heart failure. With the deepening of the understanding of the pathogenesis of heart failure and the development of evidence-based medicine, the treatment of heart failure has gradually changed from the traditional mode of "cardiotonic, diuretic, and vasodilation" to new methods such as neuroendocrine blockade and reversal of ventricular remodeling, including angiotensin converting enzyme inhibitors/angiotensin II receptor inhibitors (ACEI/ARB), aldosterone inhibitors, β-receptor blockers, etc. Although these new treatment drugs can alleviate the clinical symptoms and mortality of heart failure patients to a certain extent, they still have clinical limitations such as many adverse reactions and long-term medication, and cannot significantly improve the quality of life of patients. Relevant data show that the 5-year mortality rate of heart failure patients is still as high as over 50%.

Combination drugs are an effective method to increase drug efficacy and reduce drug side effects by combining drugs to simultaneously act on multiple signaling pathways of the disease, and are widely used in clinical treatment. Especially for diseases with complex pathogenesis such as heart failure, combination drugs are expected to become a practical treatment strategy to improve the quality of life of patients with heart failure. However, there are currently only four combination drugs for the treatment of heart failure, namely sacubitril-valsartan, lisinopril-torasemide, bisoprolol fumarate/perindopril arginine, and hydralazine hydrochloride/isosorbide dinitrate, which are far from meeting clinical drug needs. Therefore, the development of new combination drugs for anti-heart failure is of great practical significance.

### Summary of the invention

The object of the present invention is to provide a combination of an SGLT2 inhibitor and menthol, which has therapeutic advantages over single drugs and is expected to be developed as a compound drug for preventing, improving and/or treating heart diseases.

The first aspect of the present invention provides a pharmaceutical composition comprising:
(a) an SGLT2 inhibitor, a pharmaceutically acceptable salt thereof, an optical isomer thereof, a hydrate thereof, a solvate thereof, or a prodrug ester thereof;
(b) menthol, a pharmaceutically acceptable salt thereof, an optical isomer thereof, a hydrate thereof, a solvate thereof, or a prodrug ester thereof.

In another preferred embodiment, the SGLT2 inhibitor is selected from the following group: empagliflozin, dapagliflozin, canagliflozin, ipragliflozin, luseogliflozin, tofogliflozin, sotagliflozin, ertugliflozin, remogliflozin, ipragliflozin, canagliflozin, bexagliflozin, enavogliflozin, gangliflozin, sagliflozin.

In another preferred embodiment, the menthol is selected from the following group: L-menthol, D-menthol, and (DL)-menthol.

In another preferred embodiment, "pharmaceutically acceptable salt" refers to salts formed by the compound with an acid selected from a group consisting of hydrofluoric acid, hydrochloric acid, hydrobromic acid, phosphoric acid, acetic acid, oxalic acid, sulfuric acid, nitric acid, methanesulfonic acid, aminosulfonic acid, salicylic acid, trifluoromethanesulfonic acid, naphthalenesulfonic acid, maleic acid, citric acid, acetic acid, lactic acid, tartaric acid, succinic acid, oxalic acid, pyruvic acid, malic acid, glutamic acid, p-toluenesulfonic acid, naphthalenesulfonic acid, ethanesulfonic acid, naphthalenedisulfonic acid, malonic acid, fumaric acid, propionic acid, oxalic acid, trifluoroacetic acid, stearic acid, pamoic acid, hydroxymaleic acid, phenylacetic acid, benzoic acid, glutamic acid, ascorbic acid, p-aminobenzenesulfonic acid, 2-acetoxybenzoic acid and isethionic acid, etc.; or sodium salt, potassium salt, calcium salt, aluminum salt or ammonium salt, etc. formed by the compound with an inorganic base; or methylamine salt, ethylamine salt or ethanolamine salt, etc. formed by the compound with an organic base.

In another preferred embodiment, the pharmaceutical composition further comprises a pharmaceutically acceptable carrier.

"Pharmaceutically acceptable carrier" refers to: one or more compatible solid or liquid fillers or gel substances, which are suitable for human use and must have sufficient purity and sufficiently low toxicity. "Compatibility" here means that the components in the composition can be mixed with the components of the pharmaceutical composition of the present invention and with each other without significantly reducing the efficacy of the pharmaceutical composition. Examples of pharmaceutically acceptable carriers include cellulose and its derivatives (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agents (such as sodium lauryl sulfate), colorants, flavoring agents, stabilizers, antioxidants, preservatives, pyrogen-free water, etc.

In another preferred embodiment, the molar ratio of the SGLT2 inhibitor to the menthol is 1-10000: 1-10000. In another preferred embodiment, the molar ratio of the SGLT2 inhibitor to the menthol is 1-200: 1-300, preferably the molar ratio of the SGLT2 inhibitor to the menthol is 5-100: 5-250; more preferably the molar ratio of the SGLT2 inhibitor to the menthol is 1-8: 1-22, 4-6: 15-25 or 4.5-5.5: 18-22.

In another preferred embodiment, the mass ratio of the SGLT2 inhibitor to the menthol is 1-10000:1-10000. In another preferred embodiment, the mass ratio of the SGLT2 inhibitor to the menthol is 1-100:1-200, preferably the mass ratio of the SGLT2 inhibitor to the menthol is 1-20:1-100; more preferably the mass ratio of the SGLT2 inhibitor to the menthol is 1-10:1-50, 2-3:5-10, 1-2:3.

In another preferred embodiment, the pharmaceutical composition comprises empagliflozin and L-menthol.

In another preferred embodiment, the molar ratio of empagliflozin to L-menthol is 1-400:1-400.

In another preferred embodiment, the mass ratio of empagliflozin to L-menthol is 1-400:1-400.

In another preferred embodiment, the molar ratio of empagliflozin to L-menthol is 1-200:1-300, preferably the molar ratio of empagliflozin to L-menthol is 5-100:5-250; more preferably the molar ratio of empagliflozin to L-menthol is 1-8:1-22, 4-6:15-25 or 4.5-5.5:18-22.

In another preferred embodiment, the molar ratio of empagliflozin to L-menthol is 1:1-10, preferably 1:2-6, more preferably 1:4.5-5.5 or 1:4.

In another preferred embodiment, the mass ratio of empagliflozin to L-menthol is 1-100:1-200, preferably the mass ratio of empagliflozin to L-menthol is 1-20:1-100; more preferably, the mass ratio of empagliflozin to L-menthol is 1-10:1-50, 2-3:5-10 or 1-1:3.

In another preferred embodiment, the mass ratio of empagliflozin to L-menthol is 1:0.1-5, preferably 1:0.5-3, and more preferably 1:1-2 or 1:1.5.

In another preferred embodiment, the dosage form of the pharmaceutical composition is oral liquid, tablet, pill, powder, capsule, injection and granule.

In another preferred embodiment, the two drugs in the pharmaceutical composition are administered simultaneously or separately.

The pharmaceutical composition of the present invention has a protective effect on rat myocardial cell line H9c2 cells and primary myocardial cells under an injury model.

The pharmaceutical composition of the present invention can synergistically inhibit the influx of calcium ions into cells and synergistically promote fatty acid metabolism.

The second aspect of the present invention provides use of the pharmaceutical composition described in the first aspect for the manufacture of a medicament for treating heart disease.

In another preferred embodiment, the heart disease is selected from a group consisting of heart failure, coronary heart disease, myocardial infarction, myocarditis, myocardial disease, hypertensive heart disease, arrhythmia, and dilated cardiomyopathy.

In another preferred embodiment, the heart failure includes heart failure with reduced ejection fraction and heart failure with preserved ejection fraction.

It should be understood that within the scope of the present invention, the above-mentioned technical features of the present invention and the technical features specifically described below (such as examples) can be combined with each other to form a new or preferred technical solution. Each feature disclosed in the specification can be replaced by any alternative feature that provides the same, equal or similar purpose. Due to space limitations, they will not be described one by one here.

### Brief description of the drawings

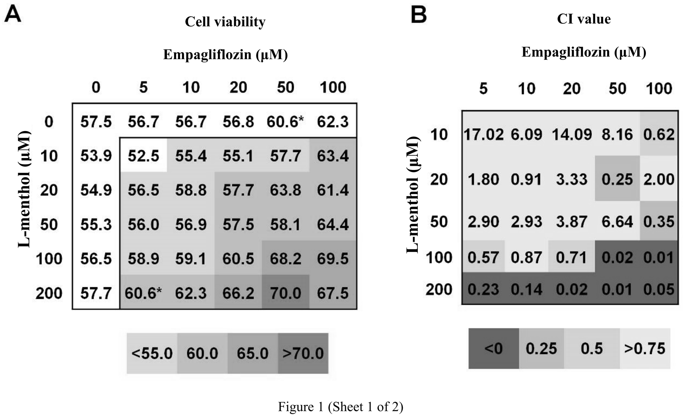

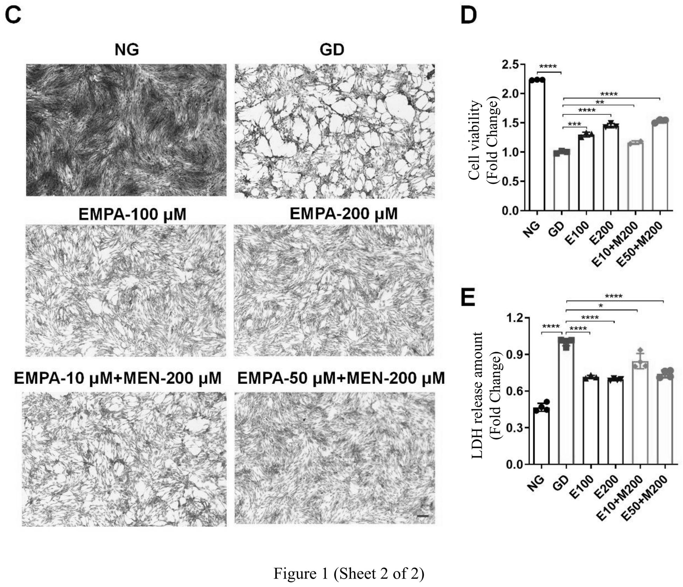

Figure 1 shows that empagliflozin and menthol synergistically protect cardiomyocytes. (A) Cell viability in the checkerboard assay; (B) Synergy index in the checkerboard assay; (C) Representative images of crystal violet staining; Scale bar, 500 µm; (D) Crystal violet staining quantification; (E) LDH release. The data are showed as mean + SD: *, p < 0.05, **, p < 0.01, ***, p < 0.001, ****, p < 0.0001.

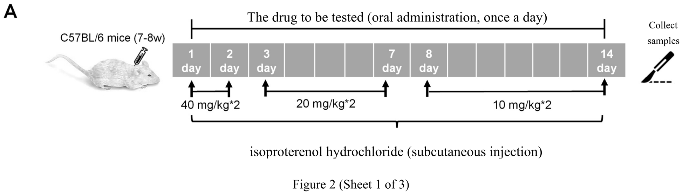

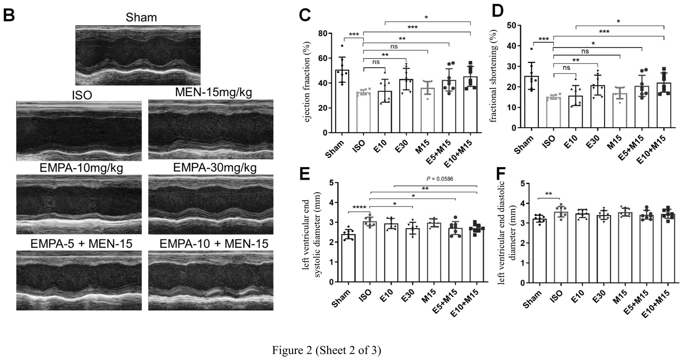

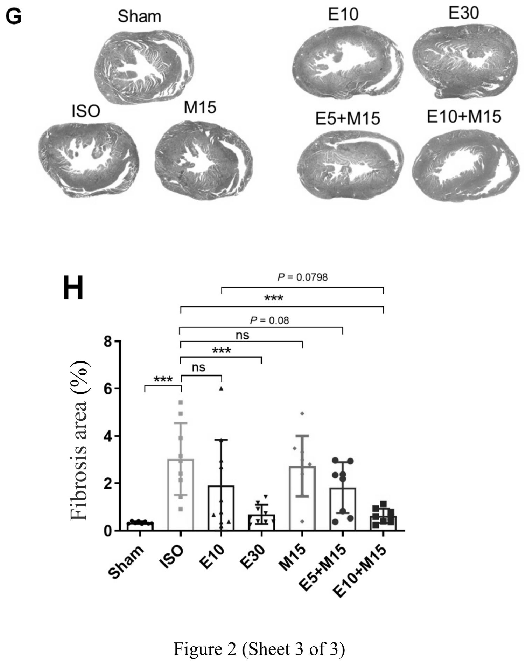

Figure 2 shows the efficacy of the combination of empagliflozin and menthol on the ISO-induced heart failure model. (A) Schematic diagram of the experimental process; (B) Representative echocardiogram in M mode; (C) Ejection fraction; (D) Fractional Shortening; (E) Left ventricular end-systolic diameter; (F) Left ventricular end-diastolic diameter; (G) Representative myocardial fibrosis map; (H) Quantification of myocardial fibrosis. Data are mean ± SD: *, p < 0.05, **, p < 0.01, ***, p < 0.001.

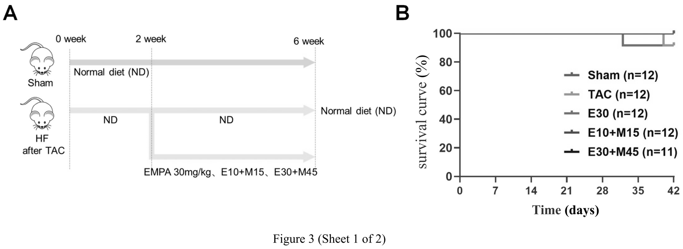

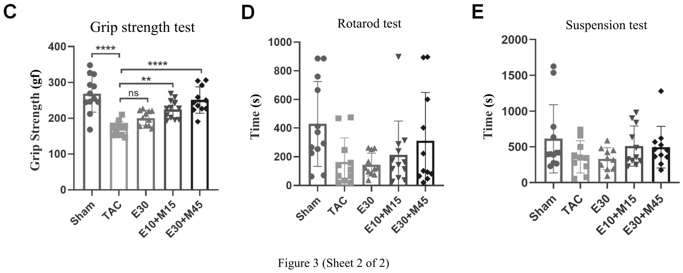

Figure 3 shows the efficacy and exercise tolerance of the combination of empagliflozin and menthol in the TAC-induced heart failure model. (A) Schematic diagram of the experimental process; (B) Survival curve; (C) Grip strength test; (D) Rotarod test; (E) Suspension test. The data are showed as mean ± SD: *, p < 0.05, **, p < 0.01, ***, p < 0.001, ****, p < 0.0001.

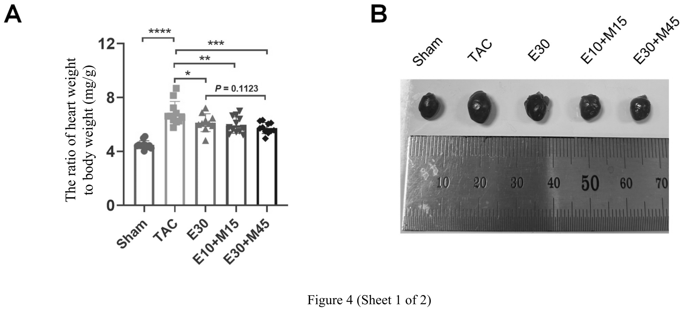

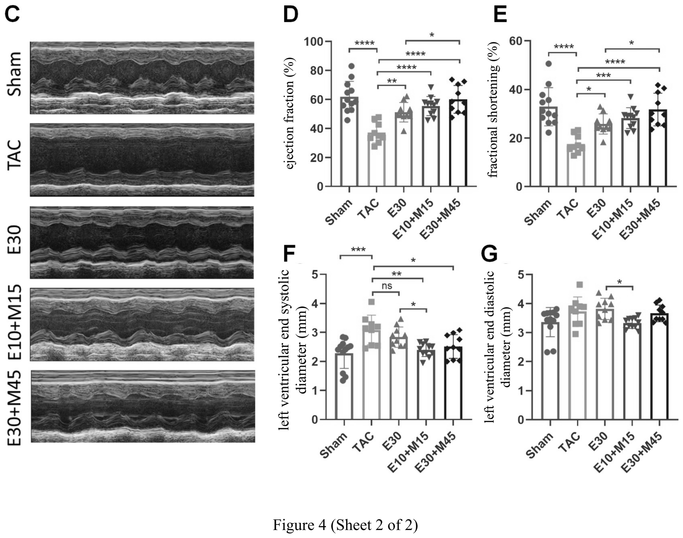

Figure 4 shows the effect of the combination of empagliflozin and menthol on cardiac function in TAC mice. (A) the heart weight to body weight ratio; (B) Representative heart; (C) Representative M-mode echocardiogram; (D) Ejection fraction; (E) Fractional shortening; (F) Left ventricular end-systolic diameter; (G) Left ventricular end-diastolic diameter. The data are showed as mean ± SD: *, p < 0.05, **, p < 0.01, ***, p < 0.001, ****, p < 0.0001.

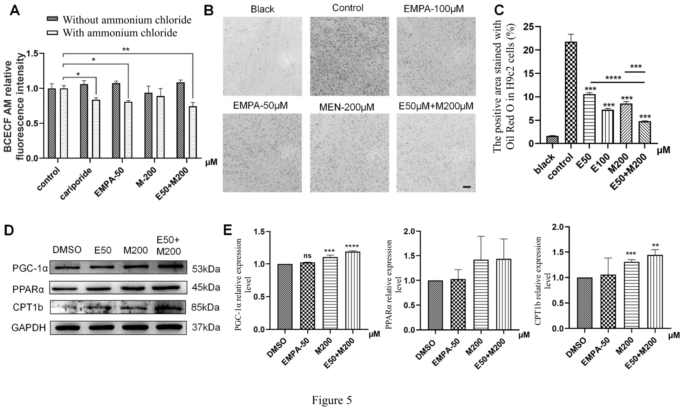

Figure 5 shows the synergistic effect of the drug combination of empagliflozin and menthol on intracellular calcium concentration and fatty acid metabolism. (A) Intracellular calcium concentration; (B) Representative Oil Red O staining images; (C) Oil Red O staining quantitative graph; (D) Representative Western blotting graph; (E) Target band quantitative graph. The data are showed as mean ± SD: *, p < 0.05, **, p < 0.01, ***, p < 0.001, ****, p < 0.0001.

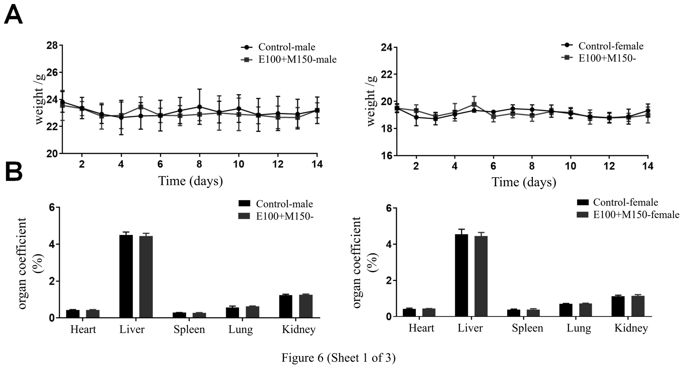

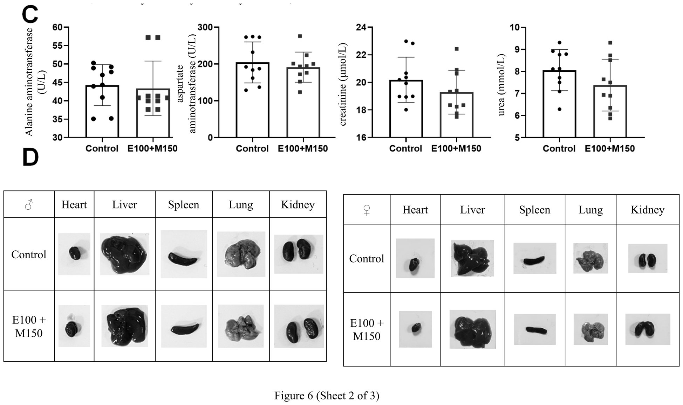

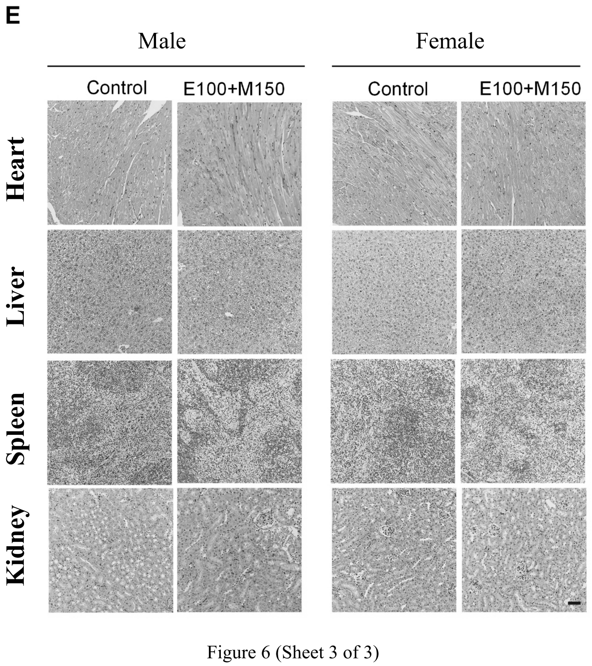

Figure 6 shows the safety of the drug combination of empagliflozin and menthol. (A) Body weight change curve; (B) Coefficients of various organs; (C) Alanine aminotransferase (ALT) content, aspartate aminotransferase (AST) content, creatinine level (CREA), urea level (UREA); (D) Representative organ appearance pictures; (E) Representative HE staining pictures of heart, liver, spleen, and kidney.

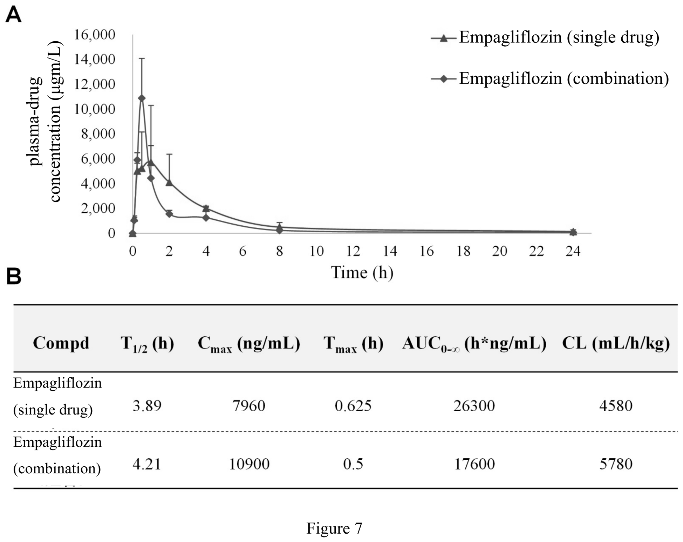

Figure 7 shows the pharmacokinetic properties of empagliflozin alone and in the presence of menthol. (A) Changes in blood drug concentration over time; (B) Main metabolic parameters.

### Detailed description

The present invention will be further described below in conjunction with specific examples. It should be understood that these examples are intended to illustrate the present invention only and are not intended to limit the scope of the present invention. The experimental methods in the following examples where specific conditions are not specified are usually performed under conventional conditions (such as those described in Sambrook et al., Molecular Cloning: Laboratory Manual (New York: Cold Spring Harbor Laboratory Press, 1989)) or according to the conditions recommended by the manufacturer. Unless otherwise stated, percentages and parts are weight percentages and weight parts.

Unless otherwise defined, all professional and scientific terms used herein have the same meanings as those familiar to those skilled in the art. In addition, any methods and materials similar or equivalent to those described herein can be applied to the methods of the present invention. The preferred implementation methods and materials described herein are for demonstration purposes only.

### Example 1 Protective effect of the pharmaceutical composition on myocardial cells

Myocardial cell death plays an important role in the pathogenesis and development of heart disease, so improving the survival of myocardial cells is beneficial to the treatment of heart disease. In this example, a glucose deprivation (GD)-induced myocardial cell injury model was used to evaluate the protective effect of the drug composition on myocardial cells. Rat myocardial cells (H9c2) were purchased from the stem cell bank of the Chinese Academy of Sciences. Myocardial cells were cultured in high-glucose DMEM (containing 10% fetal bovine serum, 1% penicillin/streptomycin) culture medium and placed in an incubator at 37°C and 5% CO₂. After the myocardial cells basically covered the cell culture dish (10 cm), the cells were digested with trypsin and planted in a 96-well plate at a cell density of 8000 cells/well, 100 µL per well. When the cells covered the bottom of the dish, the DMEM culture medium was removed and the plate was washed once with PBS. The compound was diluted to the target concentration with sugar-free and serum-free DMEM, added to the plate, and treated with GD. After GD 36 - 48h, the cell survival status was observed (the survival rate of the model group was preferably controlled at 50%, which could better highlight the cell protective effect of the compound), and the cell survival rate was detected by cck8. The cell survival rates of empagliflozin (EMPA) 0 - 100 µM, L-menthol (MEN) 0 - 200 µM and combined concentrations were tested, and the cell survival rates of single drugs and combined drugs were input into CompuSyn (http://www.combosyn.com) to calculate the synergistic coefficient. The activity data are shown in FIG. 1, panels A and B. Menthol has a good synergistic effect with empagliflozin at 100 µM and 200 µM, especially the combination of 50 µM empagliflozin and 200 µM menthol has better efficacy than that of single drugs, with a synergistic index of 0.01, which has a strong synergistic effect. In addition, the effective concentration of empagliflozin is 50 µM when used alone, and the effective concentration is reduced to 5 µM when used in combination with menthol. Note: The menthol involved in the following examples is all L-menthol.

### Example 2 Effect of the pharmaceutical composition with preferred equivalent ratio on LDH release from cardiomyocytes and crystal violet staining

To further verify the anti-heart failure efficacy of the drug composition, H9c2 cells were seeded into 24-well plates. After the cells covered the bottom of the dish, the high-glucose DMEM was removed, the plate was washed once with PBS, and empagliflozin alone and different concentrations of the drug composition were prepared with sugar-free and serum-free DMEM medium, and added to the cells for glucose deprivation treatment. For the GD group, sugar-free and serum-free DMEM medium without drugs was added. For the NG group, high-glucose DMEM containing serum was continued to be added for culture. After 48 hours of glucose deprivation treatment, 50 µL of culture medium was taken to a 96-well plate, and the release of LDH in the culture medium was detected using an LDH detection kit. The culture medium was removed, the cells were placed on ice, and washed twice with pre-cooled PBS for 3 to 5 minutes each time. The cells were fixed with pre-cooled methanol at -20°C for 10 minutes. Methanol was removed and the cells were returned to room temperature. A sufficient amount of 0.5% crystal violet staining solution was added and incubated at room temperature for 10 minutes. The crystal violet was removed and the cells were washed with clean water until they do not fade. The cells were placed in a 37 °C oven for drying, photographed under a microscope, and the staining was quantified by Image J. The larger the purple area, the more cells survived, and the more LDH was released, which indicated more cell damage.

The experiment was divided into 6 groups, namely NG group (no glucose deprivation treatment, always cultured with high-glucose DMEM containing serum), GD group (glucose deprivation treatment with glucose-free serum-free DMEM without drugs), E100 group (also known as EMPA-100 µM group, glucose deprivation treatment with glucose-free serum-free DMEM containing 100 µM empagliflozin), E200 group (also known as EMPA-200 µM group, glucose deprivation treatment with glucose-free serum-free DMEM containing 200 µM empagliflozin), E10+M200 group (i.e., EMPA-10 µM+MEN-200 µM group, glucose deprivation treatment with glucose-free serum-free DMEM containing 10 µM empagliflozin and 200 µM menthol), E50+M200 group (i.e., EMPA-50 µM+MEN-200 µM group, glucose deprivation treatment with glucose-free serum-free DMEM containing 50 µM empagliflozin and 200 µM menthol). The experimental results are shown in FIG. 1, panels C-E. Compared with the NG group, the cell survival rate in the GD group is significantly reduced, as shown by a decrease in the purple area and an increase in the amount of LDH released. empagliflozin significantly increases the cell survival rate in a dose-dependent manner at concentrations of 100 µM (EMPA-100 µM group) and 200 µM (EMPA-200 µM group), as shown by an increase in the purple quantitative area and a decrease in the amount of LDH released. The efficacy of the combination of 50 µM empagliflozin and 200 µM menthol (EMPA-50 µM+MEM-200 µM group) is equivalent to that of 200 µM empagliflozin alone, and after combined administration, empagliflozin shows significant efficacy at a concentration of 10 µM. This further indicates that the combination of empagliflozin and menthol can improve the efficacy of empagliflozin and reduce its effective concentration by at least 5 times.

### Example 3 Evaluation of the efficacy of the pharmaceutical composition in the isoproterenol-induced heart failure model

### 3.1 Experimental Principle

Isoproterenol hydrochloride is a commonly used inducer for establishing a heart failure model. Subcutaneous injection of isoproterenol hydrochloride can accelerate the heart rate of mice, cause the myocardium to continue to contract strongly, increase myocardial oxygen consumption, increase the heart load, and eventually cause heart failure.

### 3.2 Experimental methods

Preferably, 50 µM of empagliflozin and 200 µM of menthol were converted into a mass ratio of about 1:1.5 according to the molar molecular weight. The dose of empagliflozin was set at 10 mg/kg and 30 mg/kg, and the pharmaceutical composition was set as the 10 mg/kg empagliflozin + 15 mg/kg menthol group. In addition, in order to determine the extent to which the combination of menthol can reduce the effective dose of empagliflozin, a combination of menthol at a dose of 15 mg/kg and empagliflozin at a dose of 5 mg/kg was administered, resulting in the empagliflozin 5 mg/kg + menthol 15 mg/kg group.

Specifically, C57/6J male mice with an average body weight of about 21-22 g were divided into 7 groups (sham group, ISO group, empagliflozin 30 mg/kg group (E30), empagliflozin 10 mg/kg group (E10), menthol 15 mg/kg group (M15), empagliflozin 10 mg/kg + menthol 15 mg/kg group (E10+M15), empagliflozin 5 mg/kg + menthol 15 mg/kg group (E5+M15)), with 8 mice in each group.

The mice were given drugs by gavage every morning, and the solvent was DMSO (the content was less than 5%, and menthol and empagliflozin solution were mixed in equal volumes and then gavaged into the mice). The sham group and ISO group were gavaged with an equal amount of drinking water every morning.

Isoproterenol hydrochloride was injected subcutaneously twice a day, in the morning and evening, with a dose of 40 mg/kg on days 1-2, 20 mg/kg on days 3-7, and 10 mg/kg on days 8-14. The sham group was injected with an equal amount of normal saline in the morning and evening every day (FIG. 2, panel A).

On the 15th day, the Visual-Sonics Vevo 3100 small animal high-resolution micro-ultrasound imaging system was used to evaluate the cardiac structure and function of the experimental mice in each group. Three cardiac cycles were taken to measure the systolic interventricular septum thickness (IVSs), diastolic interventricular septum thickness (IVSd), left ventricular systolic inner diameter (LVIDs), left ventricular diastolic inner diameter (LVIDd), systolic left ventricular posterior wall thickness (LVPWs), and diastolic left ventricular posterior wall thickness (LVPWd), and the ejection fraction (EF, %) and fractional shortening (FS, %) were calculated. After the experiment, the mice were euthanized, and the hearts were fixed, paraffin-embedded, sliced, and Masson-stained. ImageJ was used to quantify the fibrosis area and calculate the degree of cardiac fibrosis. Graph Pad software was used for data processing and significant differences were calculated (OneWayANOVA was used between multiple groups, and Student's t test was used between two groups).

### 3.3 Experimental Results

As shown in FIG. 2, panels B-F, the cardiac function of mice in the ISO group is significantly reduced, the thickness of the left ventricular septum and the posterior wall during systole/diastole is significantly thinner, the inner diameter is enlarged, and the ejection fraction and fractional shortening drops below the normal range (the reference range for ejection fraction in normal mice is 55% - 85%, and the reference range for fractional shortening rate in normal mice is 30% - 50%). The 30mg/kg dose of empagliflozin can effectively improve the cardiac function reduction caused by isoproterenol hydrochloride, but the dose of 10mg/kg cannot significantly improve the cardiac function. Menthol slightly improved the cardiac function of mice with heart failure at a dose of 15 mg/kg, but there is no significant difference. The combination of empagliflozin and menthol can improve the cardiac function of mice in a dose-dependent manner. The cardiac function of mice in the 10 mg/kg empagliflozin + 15 mg/kg menthol group is significantly better than the efficacy of 10 mg/kg empagliflozin alone, and is equivalent to the efficacy of 30 mg/kg empagliflozin alone. 5 mg/kg empagliflozin + 15 mg/kg menthol still shows significant efficacy. In terms of myocardial fibrosis (FIG. 2, panels G and H), the area of cardiac fibrosis in the ISO group mice is significantly increased compared with that in the sham group mice. 30 mg/kg empagliflozin and 10 mg/kg empagliflozin + 15 mg/kg menthol can significantly reduce myocardial fibrosis caused by isoproterenol hydrochloride, and 5 mg/kg empagliflozin + 15 mg/kg menthol can also significantly reduce myocardial fibrosis. The above experimental results show that empagliflozin and menthol have a significant synergistic effect, which can reduce the effective dose of empagliflozin in vivo by 6 times, and the efficacy of combined administration is better than that of empagliflozin at the same dose. The combination of empagliflozin and menthol is superior to monotherapy in the treatment of heart failure.

### Example 4 Evaluation of the efficacy of the pharmaceutical composition in aortic coarctation-induced heart failure model

### 4.1 Experimental Principle

The transverse aortic constriction (TAC) model is the most commonly used disease model of chronic ventricular hypertrophy, which is used to simulate hypertrophic cardiomyopathy, heart failure, and hypertensive heart disease caused by hypertension or increased intraventricular pressure.

### 4.2 Experimental methods

Nine-week-old C57/6J male mice were divided into five groups (sham group, TAC group, 30 mg/kg empagliflozin group (E30), 10 mg/kg empagliflozin + 15 mg/kg menthol group (E10+M15), and 30 mg/kg empagliflozin + 45 mg/kg menthol group (E30+M45)). The mice in the sham group did not undergo surgical modeling, the mice in the TAC group underwent aortic arch constriction surgery but did not receive drug treatment, the mice in the E30 group underwent aortic arch constriction surgery and were treated with 30 mg/kg empagliflozin, the mice in the E10+M15 group underwent aortic arch constriction surgery and were treated with a combination of 10 mg/kg empagliflozin and 15 mg/kg menthol, and the mice in the E30+M45 group underwent aortic arch constriction surgery and were treated with a combination of 30 mg/kg empagliflozin and 45 mg/kg menthol.

Experimental process of aortic arch constriction surgery: hair was removed from the surgical area from the neck to the anterior chest of the experimental mouse, and the surgical area was disinfected with iodine. The neck and chest were opened in the midline, the muscles were bluntly separated, the incision was retracted with retractors, the thymus was dissected, and the aortic arch was exposed. The innominate artery, left common carotid artery, and left subclavian artery were found along the aortic arch. The thread was passed between the right innominate artery and the left common carotid artery, and the constriction needle was paced parallel to the aortic arch and then the thread was tied a knot. After tightening the thread, the constriction needle was slowly withdrawn, that is, quantitative constriction was formed in the aortic arch. The chest cavity was closed, the skin on the neck and chest was sutured and disinfected with iodine. Two weeks after the operation, after confirming the hypertrophy of the heart through cardiac function test, drug administration started, and continued for 4 weeks. The drug administration group was gavaged once a day in the morning, and the sham group and TAC group were gavaged with the same amount of drinking water every morning. The experimental process is shown in FIG. 3, panel A.

The survival of mice was recorded during the experiment. After 4 weeks of continuous administration, the exercise tolerance of experimental mice was evaluated by rotarod test, suspension test and grip strength test. The rotarod test evaluated the maximum tolerance time of mice at a speed of 35 rpm, the suspension test evaluated the maximum tolerance time of mice forearm suspension, and the grip strength test evaluated the maximum grip strength of mice forepaws. After the exercise tolerance test, the Visual-Sonics Vevo 3100 small animal high-resolution micro-ultrasound imaging system was used to evaluate the cardiac structure and function of experimental mice in each group. The experimental method was the same as section 3.2. After the experiment, the mice were euthanized, the hearts were weighed, the heart-body weight ratio was calculated, and then the hearts were fixed, paraffin-embedded, sliced, and Masson-stained. ImageJ was used to quantify the fibrosis area and calculate the degree of cardiac fibrosis. Graph Pad software was used for data processing and significant differences were calculated (OneWayANOVA was used between multiple groups, and Student's t test was used between two groups).

### 4.3 Experimental Results

During the experiment, one mouse died in each of the TAC group and the EMPA-30 mg/kg group, but no mouse died in the other groups (FIG. 3, panel B).

In terms of exercise tolerance (FIG. 3, panels C-E), the maximum gripping strength of mice in the TAC group is significantly lower than that of mice in the sham group, and the maximum rotarod tolerance time and the maximum tolerance time of forearm suspension are also significantly reduced, indicating that the exercise tolerance of mice in the TAC group is reduced and the motor function was weakened, which is largely related to the weakened cardiac function. Empagliflozin alone at 30 mg/kg cannot improve the exercise tolerance of TAC mice, but combined with menthol, it can significantly improve the exercise tolerance of mice in a dose-dependent manner, as shown by the significant increase in the maximum gripping strength of mice and the significant increase in the maximum rotarod tolerance time and the maximum tolerance time of forearm suspension after combined administration.

In terms of myocardial hypertrophy (FIG. 4, panels A-B), the hearts of mice in the TAC group are significantly enlarged in appearance, and the heart weight to body weight ratio is significantly increased, indicating that after TAC modeling, the mouse heart shows obvious hypertrophy. The EMPA-30mg/kg dose group of mice significantly improves myocardial hypertrophy and reduces the heart weight to body weight ratio. After combining with menthol, the effect is more significant and dose-dependent. The high-dose combination (E30+M45) has a certain advantage over the single drug EMPA-30mg/kg (P = 0.1123).

In terms of cardiac function (FIG. 4, panels C-G), the thickness of the left ventricular septum and posterior wall of the mice in the TAC group during systole/diastole is significantly thinner, the inner diameter at the end of systole and diastole is larger, and the ejection fraction and fractional shortening drop below the normal range. The 30 mg/kg dose of empagliflozin significantly improves TAC-induced cardiac dysfunction, and the combination of empagliflozin and menthol significantly improve the cardiac function of mice in a dose-dependent manner, and the efficacy of the E30+M45 dose group is significantly better than that of the single drug E30 at the same dose.

Based on the above experimental results, the empagliflozin + menthol combination of the present invention has significant advantages over empagliflozin alone in the treatment of heart failure. In addition to better efficacy in improving cardiac function and myocardial hypertrophy, the combination also has a function that empagliflozin alone does not have, namely, improving the exercise tolerance of heart failure mice, which is of great significance for improving the prognosis of heart failure patients.

### Example 5: Pharmaceutical composition synergistically reduces intracellular calcium ion concentration

### 5.1 Experimental Principle

Intracellular calcium overload is an important factor in the transition of myocardial cells from reversible to irreversible damage. Intracellular calcium overload can be seen in a variety of cardiac pathological states, such as cardiac remodeling, myocardial ischemia-reperfusion injury, viral myocarditis, dilated cardiomyopathy, arrhythmias, and congestive heart failure. The inventors have found that empagliflozin and menthol have a synergistic effect in regulating intracellular calcium. Empagliflozin can act on the sodium-hydrogen exchanger 1 (NHE1) on the myocardial cell membrane. When myocardial ischemia occurs, myocardial cell energy metabolism is impaired, and myocardial cells switch from fatty acid metabolism to sugar-free glycolysis, producing a large amount of lactic acid, which reduces the intracellular pH. As a regulatory protein for cell pH homeostasis, NHE1 can transport H⁺ out of the cell by exchanging extracellular sodium ions, resulting in an increase in the intracellular sodium ion concentration. Since myocardial cells already lack ATP at this time, the excess intracellular sodium ions cannot be excreted from the cell through the sodium-potassium exchange pump that depends on ATP, and can only be transported through the sodium-calcium exchanger that does not depend on ATP, further increasing the intracellular calcium ion concentration, causing intracellular calcium overload and affecting mitochondrial function. When mitochondrial function is impaired, the release of ROS increases, and ROS can further stimulate L-type calcium channels, promoting Ca²⁺ influx, creating a vicious cycle. Empagliflozin inhibits the activity of NHE1 and can indirectly reduce the intracellular calcium ion concentration. Menthol, as an L-type calcium channel blocker, can synergistically reduce the intracellular calcium ion concentration with empagliflozin, playing a synergistic role. In order to verify this synergistic mechanism, the intracellular calcium ion concentration was detected using the calcium ion probe Fluo4-AM.

The experiment was divided into 5 groups, including control group (no drug group), cariporide group (positive control group, 100 µM), EMPA-50 (empagliflozin, 50 µM), M-200 (menthol, 200 µM), and E50+M200 (combination of 50 µM empagliflozin and 200 µM menthol).

### 5.2 Experimental procedures

1. Detection of intracellular calcium concentration under normal intracellular environment:
   (1) Take an appropriate amount of Fluo4-AM stock solution (2 mM, BeyoTime, S1060) and dilute it to a 10 µM working solution with HBSS;
   (2) For the cells to be tested (covering the bottom of the dish), remove the culture medium and wash with HBSS three times;
   (3) Add 25 µL of Fluo4-AM working solution to each well, and then add 25 µL of compound solution to each well;
   (4) Incubate at 37 °C for 30 min for fluorescent probe loading;
   (5) Wash three times with HBSS, and add 100 µL of HBSS containing 10 mM CaCl₂ to each well. Detect quantitatively the fluorescence of Fluo4-AM using a multifunctional fluorescence microplate reader (excitation wavelength 488 nm, emission wavelength 520 nm) to determine the changes in intracellular calcium ions.

### 2. Detection of intracellular calcium concentration under acidic intracellular conditions

Since empagliflozin indirectly affects intracellular calcium concentration by inhibiting NHE1, the intracellular environment was made acidic by using ammonium chloride solution to simulate the environment during myocardial ischemia, and the effect of the administered composition on intracellular calcium ions was tested. The NHE1 inhibitor cariporide was used as a positive control.

The above step (3) was changed to add 25 µL of Fluo4-AM working solution to each well, mix the test compound with 80 mM NH₄Cl solution at a 1:1 ratio, and then add 25 µL mixed solution of the compound and NH₄Cl to each well (the final concentration of NH₄Cl was 20 mM), and the remaining steps were the same as above.

### 5.3 Experimental Results

As shown in FIG. 5, panel A, in the absence of NH₄Cl, cariporide and empagliflozin have almost no effect on intracellular calcium concentration, menthol has a weak inhibitory effect on intracellular calcium concentration, which is manifested by a decrease in fluorescence value, and empagliflozin and menthol have no obvious synergistic effect. When NH₄Cl is present, cariporide and empagliflozin can significantly reduce intracellular calcium concentration, indicating that empagliflozin does indirectly affect calcium concentration through NHE1, and the effect of menthol on calcium is basically the same as that in the absence of NH₄Cl, with a weak effect of reducing intracellular calcium concentration. The combination of menthol and empagliflozin can more significantly reduce intracellular calcium concentration, indicating that the two have a synergistic effect in reducing intracellular calcium concentration.

### Example 6 Pharmaceutical Composition Synergistically Promotes Fatty Acid Oxidation

In this example, the effects of empagliflozin and menthol on fatty acid degradation were evaluated by detecting the fatty acid content in cardiomyocytes and the content of upstream proteins of fatty acid metabolism.

### 6.1 Experimental methods

1. Oil Red O staining. H9c2 cells were seeded in 12-well plates (1×10⁵ cells per well) and cultured to 80-90% confluence. The cells were incubated with lipid medium containing 200 µM palmitic acid (Sigma-Aldrich, P0500) for 48h to induce lipid droplet accumulation. The lipid medium was removed, and the cells were cultured for 24h with medium containing 50 µM EMPA (E50, EMPA-50), 100 µM EMPA (E100), 200 µM MEN (M200), and 50 µM EMPA+200 µM MEN (E50+M200) but without palmitic acid. After the incubation, the cells were stained with Oil Red. The cells were washed 3 times with PBS and fixed with 4% paraformaldehyde at room temperature for 30 min. They were washed again with PBS 3 times and incubated with Oil Red O working solution at room temperature for 30 min. After rinsing with PBS 3 times, the cells were imaged under a microscope. The red area (positive area of lipid accumulation) was measured using Image J software package. The experiment was divided into 6 groups, black group (blank group, not treated with lipid medium and drugs), control group (control group, treated with lipid medium but not treated with drugs), E50 group (treated with lipid medium and then 50 µM EMPA), E100 (treated with lipid medium and then 100 µM EMPA), M200 (treated with lipid medium and then 200 µM menthol), E50+M200 (treated with lipid medium and then 50 µM empagliflozin + 200 µM menthol).
2. Western blotting. H9c2 cells were seeded in a 12-well plate. After 48 hours of drug treatment, the cells were harvested and the total protein in the cells was extracted with RIPA lysis buffer. After BCA quantification, 4× loading buffer was added and boiled in a 95°C metal bath for 10 minutes to denature the protein. 5-10 µg of the denatured protein sample was added to SDS-PAGE. After electrophoresis, separation, transfer, and blocking, PGC-1α, PPARα, CPT1b, and GAPDH primary antibody were added and incubated overnight at 4°C. The next day, the primary antibody was recovered, and the corresponding secondary antibody was added and incubated at room temperature for 1 hour. After washing three times, ECL chemiluminescent solution was added to the strips, and immunoblotting analysis was performed using a Tanon gel imager. ImageJ software was used to perform grayscale analysis of the immunoblot bands, GAPDH was used as an internal reference for correction, and the relative expression content of the protein was calculated. The experiment was divided into DMSO group (DMSO treatment group with content not exceeding 0.5%), E50 (50 µM EMPA treatment group), M200 (200 µM menthol treatment group), and E50+M200 (50 µM empagliflozin+200 µM menthol treatment group).

### 6.2 Experimental Results

As shown in FIG. 5, panels B and C, when empagliflozin and menthol are used alone, they can significantly reduce lipid accumulation in cardiomyocytes and promote fatty acid degradation, which is manifested by a reduction in the Oil Red O staining area. When the two drugs are combined (E50+M200), the effect of promoting fatty acid degradation is more significant, and significantly better than the effect of the two single drugs at the same concentration. Menthol alone and the combination of menthol and empagliflozin can significantly increase the expression of PCG-1α/PPARα, an upstream protein of fatty acid metabolism, and upregulate the expression of fatty acid transporter CPT1b. Empagliflozin may promote fatty acid metabolism by increasing the activity of fatty acid oxidase. The above experiments show that empagliflozin and menthol can achieve a synergistic anti-heart failure effect by synergistically promoting fat metabolism.

### Example 7 Safety Evaluation of Pharmaceutical Composition

To evaluate the safety of the drug composition, a 14-day repeated administration test was conducted on mice to assess toxicity.

### 7.1 Experimental methods

ICR mice (7-8 weeks) were purchased from Shanghai JSJ Experimental Animal Co., Ltd. The mice were divided into 2 groups (n=10, half male and half female), a control group (normal saline treatment group) and an EMPA-100mg/kg+MEN-150mg/kg treatment group (E100+M150) (100mg/kg, i.g.). The drug was administered orally once a day for 14 days. The focus of observation was mortality and changes in behavior, skin, eyes, fur, and body movement activity, and body weight was monitored daily. After 14 days, the mice were euthanized, and the heart, liver, spleen, lung, and kidney were removed for appearance evaluation, and the organ coefficient (organ weight/body weight) was calculated. The liver and kidney were fixed, paraffin-embedded, sectioned, and HE stained for histopathological analysis. Blood was collected from the orbital venous plexus, and the serum was centrifuged for biochemical analysis, including creatinine, urea, alanine aminotransferase (ALT), and aspartate aminotransferase (AST).

### 7.2 Experimental Results

The mice treated with EMPA-100mg/kg+MEN-150mg/kg have no significant changes in daily diet and body weight compared with the blank group (FIG. 6, panel A). No mouse deaths and adverse reactions are observed during the administration period. No visible lesions are observed in any organs, and there are no significant changes in organs compared to the blank group mice (FIG. 6, panel B). There were no obvious abnormalities in serum biochemical indicators (AST, ALT, Cr, UREA) (FIG. 6, panel C), and no obvious pathological damage to the heart, liver, spleen, and kidney is found in the HE staining images of mice (FIG. 6, panels D and E). The above experiments show that EMPA-100mg/kg+MEN-150mg/kg has good safety. Combined with the effective dose of EMPA-10mg/kg+MEN-15mg/kg in mice, the drug composition has a safe therapeutic window of at least 10 times, making it highly likely to be developed into a drug.

### Example 8 Evaluation of Pharmacokinetic Properties of Pharmaceutical Composition

In order to explore the pharmacokinetic properties of the combination of empagliflozin and menthol, this example tested the plasma kinetics of mice orally administered empagliflozin alone (100 mg/kg) and a combination of empagliflozin and menthol (EMPA-100 mg/kg + MEN-150 mg/kg), and the drug detected in the plasma was empagliflozin.

The experimental results (FIG. 7, panels A and B) show that after the combination of empagliflozin and menthol is used in mice, the absorption degree of empagliflozin and the drug metabolism rate in the body are basically the same as the trends of mice administered with empagliflozin alone, indicating that menthol has little effect on the absorption and metabolism of empagliflozin, and there is no adverse effect of drug-drug interaction.

## Claims

1. A pharmaceutical composition, wherein the pharmaceutical composition comprises:
(a) an SGLT2 inhibitor, a pharmaceutically acceptable salt thereof, an optical isomer thereof, a hydrate thereof, a solvate thereof, or a prodrug ester thereof;
(b) menthol, a pharmaceutically acceptable salt thereof, an optical isomer thereof, a hydrate thereof, a solvate thereof, or a prodrug ester thereof.

2. The pharmaceutical composition according to claim 1, wherein the SGLT2 inhibitor is selected from a group consisting of empagliflozin, dapagliflozin, canagliflozin, ipragliflozin, luseogliflozin, tofogliflozin, sotagliflozin, ertugliflozin, remogliflozin, ipragliflozin, canagliflozin, bexagliflozin, enavogliflozin, gangliflozin, sagliflozin.

3. The pharmaceutical composition according to claim 1, wherein the menthol is selected from a group consisting of L-menthol, D-menthol, and (DL)-menthol.

4. The pharmaceutical composition according to claim 1, wherein the pharmaceutical composition further comprises a pharmaceutically acceptable carrier.

5. The pharmaceutical composition according to claim 1, wherein the molar ratio of the SGLT2 inhibitor to the menthol is 1-10000: 1-10000; or the mass ratio of the SGLT2 inhibitor to the menthol is 1-10000:1-10000.

6. The pharmaceutical composition according to claim 1, wherein the dosage form of the pharmaceutical composition is oral liquid, tablet, pill, powder, capsule, injection or granule.

7. The pharmaceutical composition according to claim 1, wherein the pharmaceutical composition comprises empagliflozin and L-menthol.

8. The pharmaceutical composition according to claim 1, wherein the molar ratio of empagliflozin to L-menthol is 1-400:1-400; or the mass ratio of empagliflozin to L-menthol is 1-400:1-400.

9. Use of the pharmaceutical composition according to claim 1 for the manufacture of a medicament for treating heart disease.

10. The use according to claim 9, wherein the heart disease is selected from a group consisting of heart failure, coronary heart disease, myocardial infarction, myocarditis and myocardial disease, hypertensive heart disease, arrhythmia, and dilated cardiomyopathy.
